(19) Europäisches Patentamt
European Patent Office
Office européen des brevets

(11) EP 0 547 079 B1

(12) **EUROPEAN PATENT SPECIFICATION**

(45) Date of publication and mention of the grant of the patent:
**10.04.1996 Bulletin 1996/15**

(21) Application number: **91914884.1**

(22) Date of filing: **08.08.1991**

(51) Int. Cl.$^6$: **A61K 39/42**, A61K 47/48, A61K 51/10

(86) International application number: **PCT/EP91/01510**

(87) International publication number:
**WO 92/03165 (05.03.1992 Gazette 1992/06)**

(54) **NEW USE OF A MONOCLONAL ANTIBODY**

NEUE VERWENDUNG EINES MONOKLONALEN ANTIKÖRPERS

NOUVELLE UTILISATION D'UN ANTICORPS MONOCLONE

(84) Designated Contracting States:
**AT BE CH DE DK ES FR GB GR IT LI LU NL SE**

(30) Priority: **11.08.1990 DE 4025499**

(43) Date of publication of application:
**23.06.1993 Bulletin 1993/25**

(73) Proprietor: **SANDOZ LTD.**
**CH-4002 Basel (CH)**

(72) Inventor: **LOIBNER, Hans**
**A-1238 Vienna (AT)**

(74) Representative: **Bizley, Richard Edward et al**
**Hepworth, Lawrence, Bryer & Bizley**
**Merlin House**
**Falconry Court**
**Baker's Lane**
**Epping Essex CM16 5DQ (GB)**

(56) References cited:
**EP-A- 0 229 546** **EP-A- 0 304 663**

- **Journal of Experimental Medicine, volume 167, no. 2, 1 February 1988, (New York, US), M. Adachi et al.: "Expression of Ley antigen in human immunodeficiency virus-infected human T cell lines and in peripheral lymphocytes of patients with acquired immune deficiency syndrome (AIDS) and AIDS-related complex (ARC)", pages 323-331, see the abstract**
- **Journal of Virology, volume 64, no. 6, June 1990, (Baltimore, US), J.E.S. Hansen et al.: "Inhibition of human immunodeficiency virus (HIV) infection in vitro by anticarbohydrate monoclonal antibodies: peripheral glycosylation of HIV envelope glycoprotein gp120 may be a target for virus neutralization", pages 2833-2840, see the abstract and table 1**
- **Fundamental Immunology, Raven Press, 1985, 2nd Edn, p681**
- **Immunology, Immunopathology and Immunity, Harper & Row, 1908, 3rd Edn, p187**

EP 0 547 079 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Rank Xerox (UK) Business Services
2.10.4/3.4

## Description

### FIELD

The invention relates to a new use of a monoclonal antibody. It concerns the use of a monoclonal antibody BR55-2 or a fragment thereof having the specificity of monoclonal antibody BR55-2, or a variant thereof, in the treatment of HIV infections, especially of AIDS (acquired immunodeficiency syndrome).

### BACKGROUND

The use of monoclonal antibodies (MAbs) in therapeutic applications is gaining increasing acceptance.

Hybridoma technology allows the production and isolation of monoclonal antibodies with well-defined binding affinity against different antigens expressed on the surface of cells. Tumour cells and virally infected cells express characteristic antigens which are not found on normal or, respectively, uninfected cells. Therefore MAbs directed against such antigens can be used in diagnosis and therapy. In therapy selective destruction of infected cells is a main goal. This can be achieved with MAbs which activate the natural effector functions after binding to the target cell and therefore cause cell lysis. Cell destruction is effected by activation of the human complement system and activation of different human effector cells (e.g. monocytes, macrophages, natural killer cells, lymphocytes, granulocytes).

A further possibility is the use of MAbs as targeting vehicle for cytotoxic substances. These destroy the target cells after binding. Complete MAbs or fragments of MAbs having the same specificity, or variants thereof, e.g. coupled to a cell poison such as ricin, a synthetic cytotoxic substance or a radionuclide, can be used.

A. Masakazu et al J. Exp. Med. 167 (1988) 323-331 report that HIV-infected T-cells bind to an IgM antibody termed BM-1. This antibody is stated to be specific for the $Le^y$ determinant. EP 304663 reports that the $Le^y$ determinant is present on HIV-infected T-cells and proposes a method for using the IgM BM-1 antibody for detecting such cells. J.-E. S. Hansen et al. J. Virol. 64 (1990) 2833-2840 report that pretreatment of HIV with the IgM BM-1 antibody reduces infectivity. It is also reported therein that treatment of HIV-infected cells with the antibody reduces syncytia formation. M. Blaszczyk-Thurin et al. J. Biol. Chem. 262 (1987) 372-379 report that an antibody designated BR55-2 is specific for the $Le^y$ determinant.

The group of monoclonal antibodies constituted by BR55-2 and fragments thereof having the same specificity and their variants, is disclosed in e.g. Wistar EP 285059, M. Blaszczyk-Thurin et al., J. Biol. Chem 262 (1987) 372-379, or Z. Steplewsky et al., Hybridoma 9 (1990) 201-210. These publications also disclose their preparation and their use in the detection and therapy of, basically, adenocarcinomas and similar tumours. The BR55-2 class of antibodies bind to a carbohydrate antigen which is expressed on the surface of tumour cells and specifically recognizes the difucosyl Lewis blood group antigens Y-6 and B-7-2 normally associated with adenocarcinomas. These murine IgG antibodies (IgG3, IgG2a, IgG1 and IgG2b) depending on their isotype after binding to the target tumour cells very strongly activate human effector functions and destroy these cells.

### SUMMARY OF THE INVENTION

It has now been found that, surprisingly, the above BR55-2 and BR55-2-related antibodies have an excellent inhibitory activity in HIV infections. Therapeutically useful are especially such IgG isotypes which activate human effector functions and selectively mediate the destruction of HIV-infected, Y-antigen positive cells, in particular IgG3 and IgG2a.

The invention thus comprises use of a monoclonal antibody or a fragment thereof for the manufacture of a medicament for the treatment of HIV infections wherein the monoclonal antibody or fragment:
recognizes the antigen recognized by monoclonal antibodies produced by hybridomas ATCC HB 9324 and ATCC HB 9347;
has IgG isotype; and
is capable of killing HIV-infected T-cells

It further includes a pharmaceutical composition which comprises as active agent a monoclonal antibody or fragment thereof together with a pharmaceutically acceptable carrier or diluent, for use in the treatment of HIV infections wherein the monoclonal antibody or fragment:
recognizes the antigen recognized by monoclonal antibodies produced by hybridomas ATCC HB 9324 and ATCC HB 9347;
has IgG isotype; and
is capable of killing HIV-infected T-cells.

It further comprises a process for the manufacture of a medicament for use in the treatment of HIV infections which comprises mixing a monoclonal antibody or fragment thereof with a pharmaceutically acceptable carrier or diluent wherein the monoclonal antibody or fragment:
recognizes the antigen recognized by monoclonal antibodies produced by hybridomas ATCC HB 9324 and ATCC HB

9347;
has IgG isotype; and
is capable of killing HIV-infected T-cells.

In a further aspect the invention concerns the use of a monoclonal antibody BR55-2 or a fragment thereof having specificity of monoclonal antibody BR55-2, or a variant thereof, as targeting vehicle for the transport of cytotoxic substances such as ricin, synthetic cytotoxic agents and radionuclides.

## DETAILED EXPLANATION

The new use is based on the following findings:

### 1. Binding of BR55-2 antibodies to HIV-infected human T cell lines

The Lewis Y carbohydrate antigen recognized by antibodies BR55-2 is significantly expressed on HIV-infected human T cell lines. This can be shown in binding studies of the BR55-2 antibodies with e.g. cell lines HUT 78 and PALL in a cell ELISA or indirect immunofluorescence assay. Results are shown in Table 1 and Figure 2.

### 2. Complement-dependent lysis of HIV-infected human T cell lines mediated by antibodies BR55-2

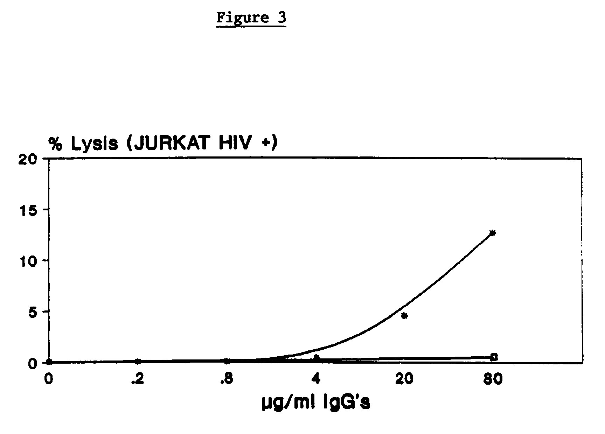

After binding to HIV-infected human T cell lines and depending on their isotype, antibodies BR55-2 activate human complement, which in turn destroys the target cells. The source of the complement to be used in such tests can be human plasma or serum. The destruction of target cells can be measured by the release of previously incorporated $^{51}Cr$ or detected by direct observation in an inverted microscope. The results from such complement-mediated cytolysis experiments using HIV-infected T cell lines JURKAT and HUT 78 are shown in Table 2 and Figure 3. It can be seen that antibodies BR55-2 mediate lysis of HIV-infected human T cell lines by activation of human complement.

The above results demonstrate the selective lytic effect of BR55-2 antibodies as defined above against HIV-infected human T cells.

Variants of BR55-2 are e.g. chimeric antibodies, humanized antibodies, class-switch variants, labelled antibodies and immunoconjugates. Fragments are e.g. Fab and $F(ab')_2$ fragments. Variants and fragments can be prepared in accordance with known methods, e.g. as described in EP 285059. Labeling may e.g. be effected with a radioisotope, a drug, an immunomodulator, a biological response modifier, a lectin or a toxin.

Preferred is intact BR55-2, especially of IgG3 isotype.

The term "a monoclonal antibody BR55-2 or a fragment thereof having the specificity of monoclonal antibody BR55-2, or a variant thereof" means a monoclonal antibody, antibody fragment or antibody variant recognizing the antigen recognized by the monoclonal antibodies produced by deposited **hybridomas BR55.2** (BR55-2/IgG3) and **BR55.2S2a** (BR-55-2/IgG2a).

These hybridomas are deposited with the American Type Culture Collection, Rockville, MD 20852, USA, **under the provisions of the Budapest Treaty**, under deposit numbers **ATCC HB 9324** and, respectively, **ATCC HB 9347**.

Antibodies BR55-2 are very well tolerated. Side effects are dependent on dosage, duration of administration and isotype of antibody administered. Following infusion of 50 mg to 100 mg BR55-2/IgG3 only moderate nausea and vomiting is observed in most patients.

Insofar as their availability or preparation is not specifically mentioned herein, the reagents and materials used in the operation of the present invention are known and available or may be prepared or equivalents thereof may be prepared in conventional manner.

In view of their property of being internalized antibodies BR55-2 are particularly suitable for targeting of toxins and radioisotopes:

1. BR55-2 antibodies and fragments and derivatives as defined above can be used as a vehicle for the transport of cytotoxic substances. Such fragments are prepared by enzymatic cleavage using e.g. pepsin.

Cleavage to the $F(ab')_2$ fragment may e.g. be carried out in the following manner: 0.5 mg/ml of BR55-2/IgG3 in sodium acetate buffer pH 4.8 are treated with pepsin (BR55-2/pepsin 50:1) at 37°C. After 30 minutes an analytical HPLC run on hydroxyapatite shows complete reaction. So cleavage is stopped after 50 minutes by titration with 1 M NaOH to pH 7. Then the solution is dialysed for 2 hours against 0.01 M sodium phosphate buffer pH 6.8. Further purification steps may be carried out using a preparative HPLC system. The dialysed sample is diluted with the same volume of pyrogen-free water and injected into a hydroxyapatite column (total volume 160 ml). Then the column is washed with eluent A (0.01 M sodium phosphate buffer pH 6.8, 0.01 mM calcium chloride) until the UV absorption is constant. The $F(ab')_2$ fragment is eluted using a linear gradient from 2 to 100 % of eluent B (0.3 M sodium phosphate buffer pH 6.8; 0.01 mM calcium chloride) over 30 minutes. The peak volume of the desired protein is 27 ml. The product of the first purification step is dialysed against buffer A overnight and divided into 3 portions.

Each portion (23 ml) is rechromatographed in a separate run. After injection of the sample the column is washed with eluent A until UV absorption is constant. Then the $F(ab')_2$ fragment is eluted using a linear gradient from 0 to 50 % over 20 minutes. The collected peaks of the 3 separations are combined and dialysed against phosphate buffered saline (without sodium azide) overnight (two buffer exchanges). The final product is sterile filtered into a pyrogen-free sterile sealed flask. The $F(ab')_2$ fragment obtained has a purity of > 99 % (HPLC).

2. For radioimmunotherapy it is necessary to successfully attach a radioisotope to the antibody so that the resultant product is stable and yet retains the ability to bind to the target antigen. Until recently iodination was the most common method, but there has been a rapid development of a variety of methods for attaching metallic isotopes to antibodies by the use of bifunctional or heterobifunctional chelating agents covalently bound to antibodies (metals cannot be attached directly to antibodies). While bifunctional chelates can be designed to attach to a variety of positions on the antibody molecule, the majority binds to lysine residues, which are present in abundance. The final complexing of the metallic ion is accomplished immediately prior to use and can be performed simply without further loss of antibody activity. BR55-2 has been conjugated using the hydroxysuccinimide ester of DTPA because conjugates obtained thereby generally retain the antibody-binding properties better than when the cyclic anhydride method is used:

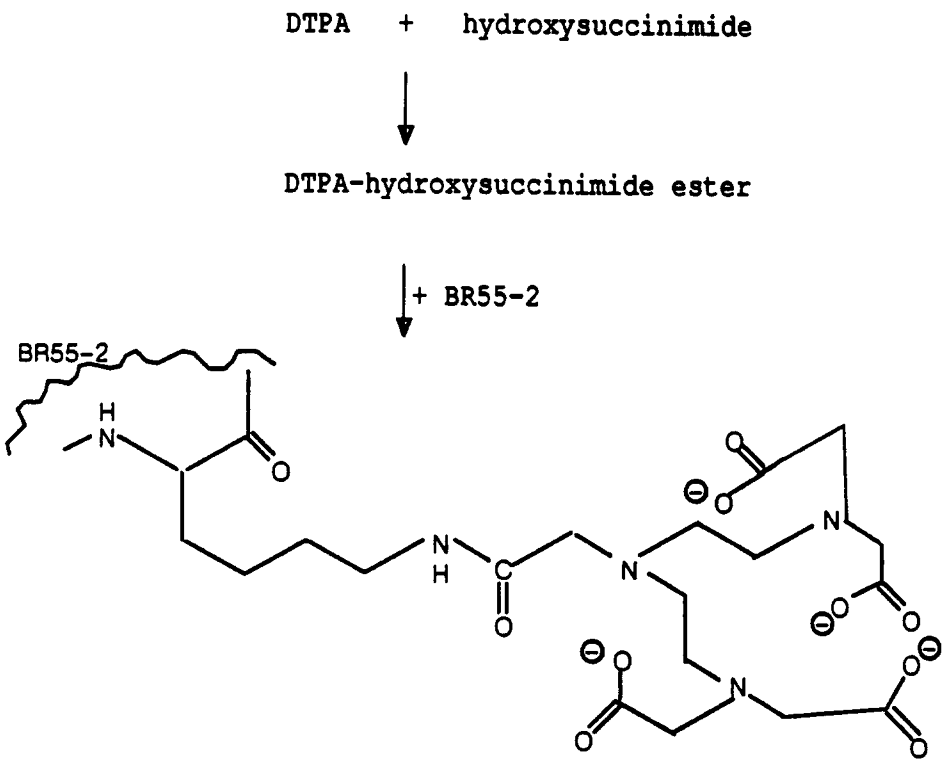


conjugate

Conjugation can be carried out e.g. in the following manner: 300 ml (6.5 µmole; 3.4 mg/ml in PBS) of antibody solution are adjusted to pH 8.0-8.2 with NaOH. 1.9 ml of DTPA-hydroxysuccinimide ester is added dropwise to the reaction mixture (pH adjusted with NaOH 0.1 N) and stirred for 2 hours. Isolation and purification of the conjugate is carried out in conventional manner.

3. The respective binding of BR55-2, the DTPA conjugate and its $^{111}In$-labeled derivative to the Y antigen expressing human mammacarcinoma cell line SKBR-5 are compared in a cell ELISA by the following procedure: after treatment of Nunc™ immunoplates II with poly-L-lysine the SKBR-5 cells are pipetted into the wells ($4 \times 10^6$ cells/ml) and fixed with glutardialdehyde. Free aldehyde groups are saturated with 1 % BSA. Sample incubation (concentrations starting with 100 µg/ml down to 0.02 µg/ml) is carried out for 1 hour at 37°C. Rabbit anti-mouse IgG/peroxidase is incubated for 40 minutes at 37°C, the binding of the MAb to the cells is determined using the peroxidase substrate o-phenylendiamine dihydrochloride. For both the conjugate and the $^{111}In$-labeled derivative no significant difference is found in comparison to unconjugated BR55-2 (Figure 1).

**EXPLANATION OF THE FIGURES:**

**Figure 1:** Binding of MAb and conjugates to cell line SKBR5 Y-antigen:
1 = BR 55-2/IgG3
2 = BR 55-2/IgG3 - DTPA conjugate
3 and 4 = $^{111}$In-labelled derivative of 2 (395 µCi/mg protein) (double determination)

**Figure 2:** Binding of BR 55-2/IgG3 to infected and non-infected cell line HUT 78 (cell ELISA)
* = HIV-infected HUT 78
□ = non-infected HUT 78

**Figure 3:** Complement-dependent cytotoxicity to HIV-infected JURKAT cell line mediated by BR 55-2/IgG3:
* = BR 55-2/IgG3
□ = MIgG

The following Examples illustrate the invention. All temperatures are in degrees Centigrade. The abbreviations have the following meanings:

BSA: bovine serum albumin

CDC: complement-dependent cytolysis

DTPA: diethylenetriaminepentaacetic acid

ELISA: enzyme-linked immunosorbent assay

FCS: fetal calf serum

FITC: fluorescein isothiocyanate

HIV: human immunodeficiency virus

MAb: monoclonal antibody

MIgG: polyclonal mouse IgG antibody

PBS: phosphate-buffered saline

RPMI: Roswell Park Memorial Institute

SDS: sodium dodecyl sulfate

The materials referred to in the Examples are as follows: Cell lines HUT 78, JURKAT, PALL: human T cell lines

Medium A:
RPMI 1640 + 2 g/l $NaHCO_3$
100 U/ml penicillin G
100 µg/ml streptomycin sulfate
4 mM glutamine
10 % FCS (heat-inactivated, γ-globulin-free)

PBS complete:
8.0 g NaCl
0.2 g $KH_2PO_4$
0.2 g KCl
1.15 g $Na_2HPO_4$
0.13 g $CaCl_2 \cdot 2H_2O$
0.1 g $MgCl_2 \cdot 6H_2O$
ad 1 l with dist. $H_2O$

| | |
|---|---|
| PBS deficient: | 138.0 mM NaCl<br>1.5 mM KOH<br>2.7 mM KCl<br>6.5 mM $Na_2HPO_4$<br>pH 7.2 |
| $Na_2{}^{51}CrO_4$: | 1 mCi/ml. |

**Example 1: Binding of BR55-2/IgG3 antibodies to HIV-infected or non-infected HUT 78 cell line**

(Cell ELISA)

Cell line HUT 78 is infected by suspending the cells ($10^6$ cells/ml) in a suspension of virus HTLV IIIb. Adsorption is allowed for 2 hours at 37°. Then the cells are spun down, the inoculum is removed and the cells are resuspended at $10^5$ cells/ml. The concentration of viral p24 antigen is determined in the supernatants by means of a commercial ELISA kit and serves as parameter for virus production.

Microtiter plates are pretreated with poly-L-lysine hydrobromide (20-30 kD; 20 µg/ml in PBS deficient; 100 µl/well; 30 minutes; room temperature), washed twice with PBS deficient (200 µl/well) and then incubated overnight at 4° with a suspension of the HUT 78 cell line (HIV-infected or non-infected) at a concentration of 4 x $10^6$ cells/ml (50 µl of cell suspension/well; medium A). After centrifugation and removal of the supernatant the cells are fixed with 50 µl of glutardialdehyde (0.1 % in physiological saline) per well for 5 minutes at room temperature, centrifuged, the supernatant is removed, the cells resuspended in 200 µl/well of PBS deficient/1 % BSA/0.1 % $NaN_3$ and left for 1 hour at room temperature. After removal of the supernatants and washing twice with 200 µl of PBS/Tween 20™ (0.05 %) per well, BR55-2/IgG3 is incubated for 1 hour at 37° (dilutions in PBS deficient). 20 µg/ml is chosen as the highest concentration for the determination of antibody binding to the test cells. Unbound antibody is washed out with 2 x 100 µl of ice-cold PBS/Tween 20 TM (0.05 %) per well and rabbit anti-mouse IgG-peroxidase 1:1000 in PBS deficient/2 % FCS is added. After incubation for 40 to 60 minutes at 37° the wells are washed thrice with the above PBS/Tween 20™ solution and then 100 µl of the following substrate solution are added to each well: 40 mg o-phenylenediamine dihydrochloride, 100 ml of staining buffer, 20 µl of $H_2O_2$ (30 %). After about 5 minutes colour development is stopped by addition of 50 µl of 4N $H_2SO_4$ per well. Binding of the antibody to HIV-infected and non-infected HUT 78 cells is determined by measuring extinction at 492 nm (calibration is at 620 nm). All washing steps are effected by cell centrifugation and resuspension. The results are shown in Figure 2 and indicate that the Lewis Y carbohydrate antigen recognized by BR55-2 is significantly expressed only on HIV-infected HUT 78 cells.

**Example 2: Binding of BR55-2/IgG3 antibodies to HIV-infected or non-infected PALL cell line**

(Immunofluorescence assay)

In a commercially available immunofluorescence assay kit (Waldheim, IFA-kit) HIV-infected and non-infected PALL cells are fixed on slides. After incubation with 1 % BSA for 30 minutes at 37° the slides are incubated with BR55-2/IgG3 in PBS deficient in appropriate concentrations for 1 hour at 37°. Irrelevant mouse-IgG serves as control. 100 µg/ml is chosen as the highest concentration for BR55-2/IgG3, 1000 µg/ml for the irrelevant mouse-IgG. Unbound antibody is washed out thrice with PBS deficient and goat anti-mouse IgG-FITC 1:30 in PBS deficient/2 % FCS is added. After incubation for 30 minutes at 37°, washing thrice with PBS deficient and embedding of the slides the immunofluorescence is observed in a fluorescence microscope. The results are shown in Table 1:

Table 1

| Antibody | Concentration (µg/ml) | PALL non-infected | PALL HIV-infected |
|---|---|---|---|
| BR55-2/IgG3 | 0 | - | - |
| | 1 | - | - |
| | 10 | - | ++ |
| | 100 | - | + |
| irrelevant mouse-IgG | 0 | - | - |
| | 1 | - | - |
| | 10 | - | - |
| | 100 | - | - |
| | 1000 | - | - |

++ = significant immunofluorescence
+ = moderate immunofluorescence
- = no immunofluorescence

The results in Table 1 show that the Lewis Y carbohydrate antigen recognized by BR55-2 is significantly expressed only on HIV-infected PALL cells.

**Example 3: Human complement-dependent cytolysis (CDC) of HIV-infected JURKAT cell lines mediated by BR55-2/IgG3**

($^{51}$Cr-release assay)

The human JURKAT T cell line is infected by suspending the cells ($10^6$ cells/ml) in a suspension of virus HTLV IIIb. Adsorption is allowed for 2 hours at 37°. Then the cells are spun down, the inoculum is removed and the cells are resuspended at $10^5$ cells/ml. The concentration of the viral p24 antigen is determined in the supernatants by means of a commercial ELISA kit and serves as parameter for virus production.

On the day preceding the assay the cells are transferred into fresh medium A and kept at 37°/5 % $CO_2$ in a cell culture flask.

$^{51}$Cr labelling of the target cells:

The cells are collected from the culture flask and incubated at a concentration of 5 x $10^6$ cells in 800 µl of medium A at 37°/ 5 % $CO_2$ for 2 hours with 100 µCi $Na_2{}^{51}CrO_4$. The cells are then washed with medium A to remove the excess $^{51}$Cr, resuspendend in fresh medium A and their concentration is adjusted to 2 x $10^5$ to 3 x $10^5$ cells/ml.

CDC:

100 µl aliquots of this suspension of target cells are pipetted into wells of microtiter plates and 50 µl aliquots of the antibody solution diluted to the desired concentration in PBS deficient are added. Then 100 µl aliquots of a human serum (1:2 dilution in medium A; final dilution of human serum 1:5) are added per well and the cells are incubated overnight at 37°/5 % $CO_2$. The supernatants are harvested with a Skatron-Harvesting-Press and counted in a γ-counter. This yields the value for the experimental release.

For determination of total $^{51}$Cr release the cells are treated as above but with the human serum replaced by a solution of 2 % SDS, 50 mM $Na_2CO_3$ and 10 mM EDTA. The value for spontaneous $^{51}$Cr release is obtained by replacing the human serum with medium A and the antibody solution with 50 µl PBS.

After counting the result is computed as follows:

$$\%\ \text{lysis} = \frac{\text{(experimental release minus spontaneous release) x 100}}{\text{total release minus spontaneous release}}$$

The results are shown in Figure 3 and indicate that BR55-2/IgG3 mediates the lysis of HIV-infected JURKAT cells by complement activation.

**Example 4: Human complement-dependent cytolysis (CDC) of HIV-infected and non-infected HUT 78 cell line mediated by BR55-2/IgG3**

(Microscopical detection)

The human T cell line HUT 78 is infected as described under Example 1. On the day preceding the assay the HIV-infected and non-infected HUT 78 cells are transferred into fresh medium A and kept at 37°/5 % $CO_2$ in cell culture flasks (3 x $10^5$ cells/ml). 100 µl aliquots of the suspension of the respective target cells are pipetted into wells of a Lab-tek™ and 50 µl of the antibody solution diluted to the desired concentration in PBS deficient are added. Then 100 µl aliquots of a human serum A (1:2 dilution in medium A; final dilution of human serum 1:5) or medium A are added per well and the cells are incubated for 2 hours at 37°/5 % $CO_2$. Morphological changes of the target cells are detected by using an inverted microscope. The results are shown in Table 2:

Table 2

| Antibody | Concentration (µg/ml) | HUT 78 non-infected | HUT 78 HIV-infected |
|---|---|---|---|
| BR55-2/IgG3 in the presence of human serum | 20 | - | + |
| BR55-2/IgG3 in the absence of human serum | 20 | - | - |
| control without antibody in the presence of human serum | 0 | - | - |
| + = cell lysis<br>- = no morphological changes | | | |

The results in Table 2 above show that with cell line HUT 78 BR55-2/IgG3 mediates the lysis of HIV-infected HUT 78 cells by complement activation.

In view of the above experimental results monoclonal antibodies BR55-2 and fragments thereof having the specificity of BR55-2, and variants thereof, are thus indicated for use in the treatment of HIV infections, especially of AIDS.

Since they show a restricted binding specificity associated with a lack of cross-reactivity to related antigens expressed on blood cells, e.g. erythrocytes, they are particularly suited for therapeutic use in humans.

For the above-mentioned use the dosage will, of course, vary depending upon e.g. the compound employed, the subject patient's age, the stage of disease, the mode of administration or the treatment desired, and can be determined by the specialist in each individual situation. It will also vary when the antibodies are used in combination with chemotherapeutic agents or immunostimulators, e.g. reverse transcriptase inhibitors. Administration is e.g. parenteral by injection or infusion. The dosage administered is e.g. of from about 25 mg to about 100 mg BR55-2 antibody as defined above every second day, preferably by slow intravenous infusion.

**Claims**

**1.** Use of a monoclonal antibody or a fragment thereof for the manufacture of a medicament for the treatment of HIV infections wherein the monoclonal antibody or fragment:
recognizes the antigen recognized by monoclonal antibodies produced by hybridomas ATCC HB 9324 and ATCC HB 9347;
has IgG isotype.

**2.** A pharmaceutical composition which comprises as active agent a monoclonal antibody or fragment thereof together with a pharmaceutically acceptable carrier or diluent, for use in the treatment of HIV infections wherein the monoclonal antibody or fragment:
recognizes the antigen recognized by monoclonal antibodies produced by hybridomas ATCC HB 9324 and ATCC HB 9347;
has IgG isotype.

**3.** A process for the manufacture of a medicament for use in the treatment of HIV infections which comprises mixing a monoclonal antibody or fragment thereof with a pharmaceutically acceptable carrier or diluent wherein the nonoclonal antibody or fragment:

recognizes the antigen recognized by monoclonal antibodies produced by hybridomas ATCC HB 9324 and ATCC HB 9347;
has IgG isotype.

4. A use according to claim 1, composition according to claim 2, or process according to claim 3, wherein the isotype is IgG3.

5. A use according to claim 1, composition according to claim 2, or process according to claim 3, wherein the antibody is an $F(ab')_2$ fragment.

6. A use according to claim 1, whever the medicament is adapted to serve as a targeting vehicle for the transport or cytotoxic substances, synthetic cytotoxic agents or radionuclides.

7. A use according to claim 1, composition according to claim 2, or process according to claim 3, wherein the monoclonal antibody is an antibody produced by hybridoma ATCC HB 9324 or ATCC HB 9347 or a humanized version thereof.

## Patentansprüche

1. Verwendung eines monoklonalen Antikörpers oder eines Fragments hiervon zur Herstellung eines Arzneimittels zur Behandlung von HIV-Infektionen, wobei der monoklonale Antikörper oder dessen Fragment ein Antigen erkennt, welches durch monoklonale Antikörper erkannt wird, die von den Hybridomas ATCC HB 9324 und ATCC HB 9347 produziert werden, und vom IgG-Isotop ist.

2. Eine Arzneimittelzusammensetzung, umfassend als ein aktives Mittel zur Verwendung bei der Behandlung von HIV-Infektionen einen monoklonalen Antikörper oder dessen Fragment zusammen mit einem pharmazeutisch verträglichen Träger oder einem Verdünnungsmittel, wobei der monoklonale Antikörper oder dessen Fragment ein Antigen erkennt, welches durch monoklonale Antikörper erkannt wird, die von den Hybridomas ATCC HB 9324 und ATCC HB 9347 produziert werden, und vom IgG-Isotop ist.

3. Ein Verfahren zur Herstellung eines Arzneimittels für die Verwendung bei der Behandlung von HIV-Infektionen, umfassend das Vermischen eines monoklonalen Antikörpers oder eines Fragments hiervon mit einem pharmazeutisch verträglichen Träger oder Verdünnungsmittel, wobei der monoklonale Antikörper oder dessen Fragment ein Antigen erkennt, welches durch monoklonale Antikörper erkannt wird, die von den Hybridomas ATCC HB 9324 und ATCC HB 9347 produziert werden, und vom IgG-Isotop ist.

4. Verwendung nach Anspruch 1, eine Zusammensetzung nach Anspruch 2 oder ein Verfahren nach Anspruch 3, wobei der Isotyp IgG3 ist.

5. Verwendung nach Anspruch 1, eine Zusammensetzung nach Anspruch 2 oder ein Verfahren nach Anspruch 3, wobei der Antikörper ein $F(ab')_2$-Fragment ist.

6. Verwendung nach Anspruch 1, wobei das Arzneimittel geeignet ist als Zielträger zum Transport von zelltoxischen Substanzen, synthetischen zelltoxischen Mitteln oder Radionukliden zu dienen.

7. Verwendung nach Anspruch 1, eine Zusammensetzung nach Anspruch 2 oder ein Verfahren nach Anspruch 3, wobei der monoklonale Antikörper ein Antikörper ist, hergestellt durch die Hybridomas ATCC HB 9324 oder ATCC HB 9347 oder eine humanisierte Version davon.

## Revendications

1. Utilisation d'un anticorps monoclonal ou un fragment de celui-ci pour la fabrication d'un médicament pour le traitement des infections par le VIH, où l'anticorps monoclonal ou son fragment :
reconnaît l'antigène reconnu par les anticorps monoclonaux produits par les hybridomes ATCC HB 9324 et ATCC HB 9347 ;
a l'isotype IgG.

2. Composition pharmaceutique qui comprend, à titre d'agent actif, un anticorps monoclonal ou un fragment de celui-ci avec un support ou un diluant pharmaceutiquement acceptable, pour une utilisation dans le traitement des infections par le VIH, où l'anticorps monoclonal ou son fragment :

reconnaît l'antigène reconnu par les anticorps monoclonaux produits par les hybridomes ATCC HB 9324 et ATCC HB 9347 ;

a l'isotype IgG.

3. Procédé de fabrication d'un médicament pour une utilisation dans le traitement des infections par le VIH, qui comprend le mélange d'un anticorps monoclonal ou un fragment de celui-ci avec un support ou un diluant pharmaceutiquement acceptable, où l'anticorps monoclonal ou son fragment :

reconnaît l'antigène reconnu par les anticorps monoclonaux produits par les hybridomes ATCC HB 9324 et ATCC HB 9347 ;

a l'isotype IgG.

4. Utilisation selon la revendication 1, composition selon le revendication 2, ou procédé selon la revendication 3, où l'isotype est IgG3.

5. Utilisation selon la revendication 1, composition selon la revendication 2, ou procédé selon la revendication 3, où l'anticorps est un fragment $F(ab')_2$.

6. Utilisation selon la revendication 1, où le médicament est adapté pour servir de véhicule de ciblage pour le transport de substances cytotoxiques, d'agents cytotoxiques synthétiques, ou de radionucléides.

7. Utilisation selon la revendication 1, composition selon la revendication 2, ou procédé selon la revendication 3, où l'anticorps monoclonal est un anticorps produit par les hybridomes ATCC HB 9324 ou ATCC HB 9347, ou une version humanisée de celui-ci.

Figure 1

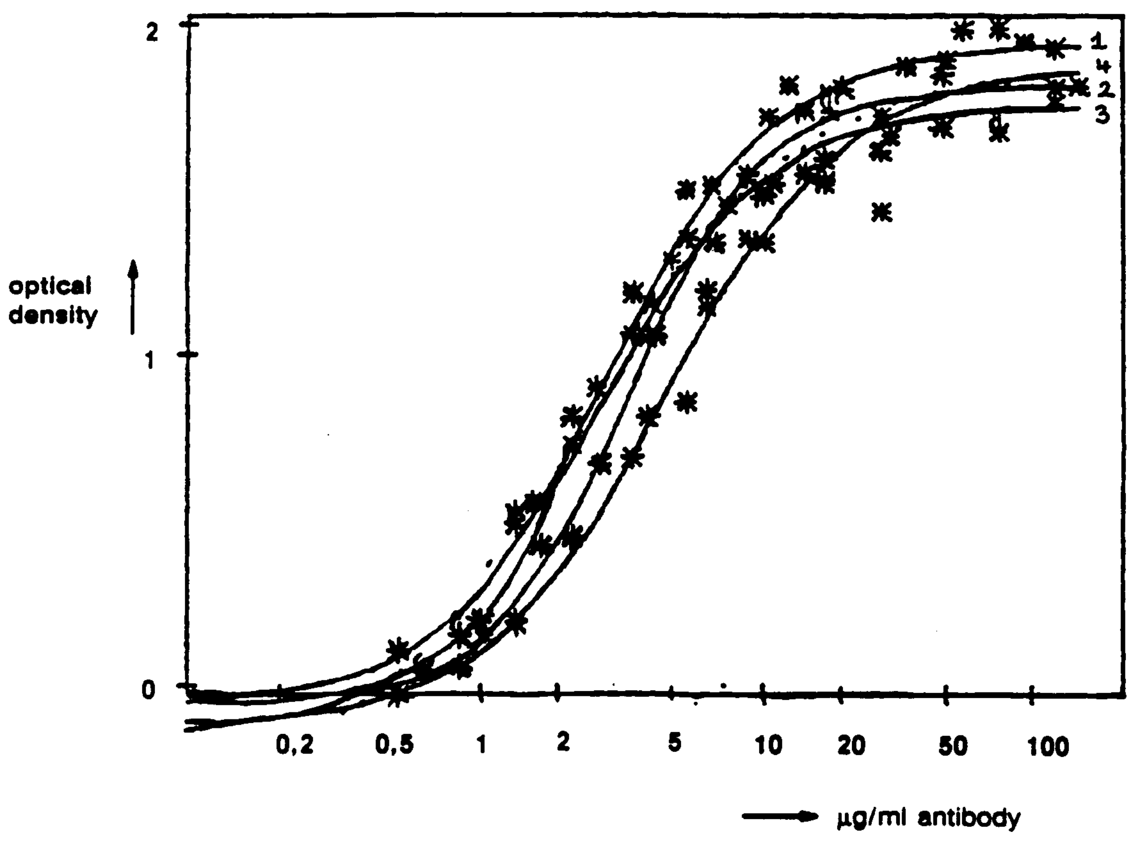

Figure 2

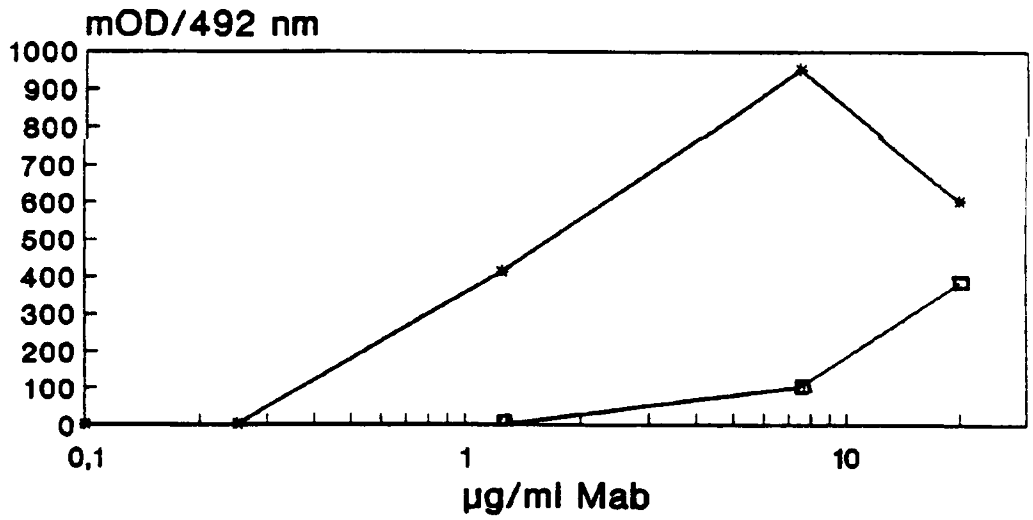
mOD/492 nm
1000
900
800
700
600
500
400
300
200
100
0
0,1
1
10
µg/ml Mab

Figure 3